# EUROPEAN PATENT SPECIFICATION

(11) **EP 0 381 182 B1**
(45) Date of publication and mention of the grant of the patent: **02.08.1995**
(21) Application number: 90101889.5
(22) Date of filing: 31.01.1990
(51) Int. Cl.: A61K 9/22

(54) **An erosion-controlled release system for active agents and a process for its preparation**
Erosionsgesteuertes Freisetzungssystem für Wirkstoffe sowie Verfahren zu dessen Herstellung
Système contrôlé par l'érosion de libération de substances actives et son procédé de préparation

(30) Priority: 31.01.1989 DD 325353; 03.10.1989 DE 3933000
(43) Date of publication of application: 08.08.1990
(73) Proprietor: IPA INTERNATIONALE PHARMA AGENTUR GMBH, 89231 Neu-Ulm (DE)
(72) Inventor: Wenzel, Udo, Prof.Dr.sc.nat., DD-4050 Halle/Saale (DD); Metzner, Jürgen, Dr.sc.med.Dr.rer.nat., DD-4020 Halle/Saale (DD); Nindel, Horst, Dr.rer.nat., DD-4020 Halle/Saale (DD); Jaeger, Halvor, Dr.med., D-7910 Neu-Ulm/Gerlenhofen (DE); Salama, Zoser B., Dr. rer.nat, D-7913 Senden/Wullenstetten (DE)
(74) Representative: VOSSIUS & PARTNER

(56) References cited:
- EP-A- 0 299 668
- WO-A-89/04673
- FR-A- 2 113 947
- US-A- 4 520 009

## Description

The invention relates to a system for erosion-controlled active agent release and to a process for its preparation. The system is suitable for various kinds of active agents and will be explained in the following using a preferred embodiment, i.e. the release of a pharmaceutical agent, as an example. If it is an oral pharmaceutical preparation, the system can preferably enable controlled release of various active agents, especially such of low solubility, from the pharmaceutical preparation. In a combination of active agents of low solubility and such of high solubility, the system can release the latter with only a slight delay, this having an advantageous effect in combination therapy.

The ideal peroral delayed release pharmaceutical preparation is such that the release of the active agent to be delayed takes place over a certain period of time, it being determined by therapeutical and physiological aspects, and the maintenance of the desired blood concentration of the active agent(s) is ensured for this period of time.

In the technology of pharmaceutics, it is known to integrate active agents into formulations in such a manner that they can be released from these formulations at a predetermined rate.

Active agents which have to be taken several times a day in therapeutic treatment, either because they are eliminated too rapidly from the organism or because a lower number of applications and the accordingly higher doses entail blood level fluctuations which cannot be tolerated, are often transformed into formulations operating with delayed release.

The known processes as described in textbooks, monographies and patent specifications (e.g. Pharmazeutische Technologie/edited by H. Sucker; Stuttgart 1978; Voigt, Lehrbuch der Pharmazeutischen Technologie, Berlin 1988) permit controlled release of the active agent according to its properties and dosage via the addition of specifically suitable hydrophilic adjuvants (e.g. gel-forming cellulose derivatives capable of swelling such as methyl cellulose, hydroxypropylmethyl cellulose, Na-carboxymethyl cellulose, polyvinyl alcohol etc.) or hydrophobic adjuvants (e.g. acylates, silicons, fats, fatty acids and their salts, polyethylene, PVC etc.). When the active agent is embedded in a hydrophilic matrix, the release thereof is controlled by the diffusion-hampering gel layer formed when the matrix comes into contact with water or digestive fluids. If said agent is embedded in hydrophobic adjuvants or surrounded thereby, it is they who form the barriers that prevent diffusion.

As a general rule, substances of high water-solubility have to be processed with adjuvants of low solubility or degradation, and substances of low water-solubility with adjuvants of high solubility or degradation.

These delayed release adjuvants are sometimes used alone, but more often with other adjuvants without which processing would not be possible. In the case of low-dose active agents, it is especially necessary to use suitable fillers which increase the amount of tablet composition but have a minimal effect on the release properties of the incorporated active agent from the standard or delaying pharmaceutical preparation. In addition to lactose, the various forms of starch known are now widely used as fillers in standard pharmaceutical preparations. Microcrystalline celluloses are especially suitable fillers for powder compression. Due to its fibrous structure, cellulose often functions as a binder, too. However, microcrystalline cellulose yields tablets with a very short disintegration period (Pharmazeutische Technologie/edited by H. Sucker; Stuttgart 1978, page 373). Thus for the person of skill in the art the use of these microcrystalline celluloses is not practical in delayed release pharmaceutical preparations. This is because they have only limited swelling ability which leads to the rapid degradation of the tablet when contacted with water or digestive fluids, but does not form a gel barrier which hampers release, such as substituted, soluble, high-swelling cellulose derivatives (methyl cellulose, propyl cellulose, hydroxypropyl cellulose, Na-carboxymethyl cellulose etc.).

There is thus a need for the provision of a solid pharmaceutical preparation and of a process for preparing a solid active agent preparation which permits the total and controlled release of the incorporated active agent or combinations of active agents for a desired, both physiologically and therapeutically practical period of time. In the preparation process, the suitable adjuvants to be used are industrially available, pharmaceutically and technologically easy to process and non-toxic.

The problem underlying the invention is to develop an erosion-controlled active agent release system and a process for the preparation thereof, according to which it is possible to prepare a delayed release form for a low solubility active agent, for combinations of low solubility active agents or for combinations of at least one low solubility active agent together with at least one high solubility active agent, having delayed release for the low solubility active agent components and, if incorporated, substantially unimpaired release for the high solubility active agent components.
This problem is solved by the invention.

Thus the subject matter of the invention is an erosion-controlled release system for pharmaceutically active agents containing at least one low solubility pharmaceutically active agent, optionally together with high solubility pharmaceutically active agents, having delayed release for the low solubility active agent components and, if incorporated, only slightly delayed release for the high solubility active agent components, characterized in that it is obtainable by formulating in a manner known per se the active agent(s) with at least one adjuvant capable of limited swelling and at least one adjuvant (swelling controller) having higher affinity to water than the adjuvant capable of limited swelling and, optionally, under the addition of further conventional adjuvants and compressing the formulation, except a formulation containing 25 % theophylline, 60 % microcrystalline cellulose and 15 % polyethylene glycol MW 20,000 and except a constant release aspirin tablet comprising from 85-95 weight percent of aspirin, from 1.5-5 weight percent of microcrystalline cellulose, from 1-10 weight percent of cellulose acetate phthalate, from 0.75-4 weight percent of a plasticizer, from 0.75-5 weight percent of corn starch, and from 0.5 to 2 percent of a lubricant.

A further subject matter of the invention is a process for preparing the erosion-controlled active agent release system which is characterised in that the active agent(s) is (are) formulated in a manner known per se and optionally compressed into tablets, this being done using at least one adjuvant capable of swelling and at least one adjuvant (swelling controller) having higher affinity to water than the adjuvant capable of swelling, optionally under the addition of further additional adjuvants.

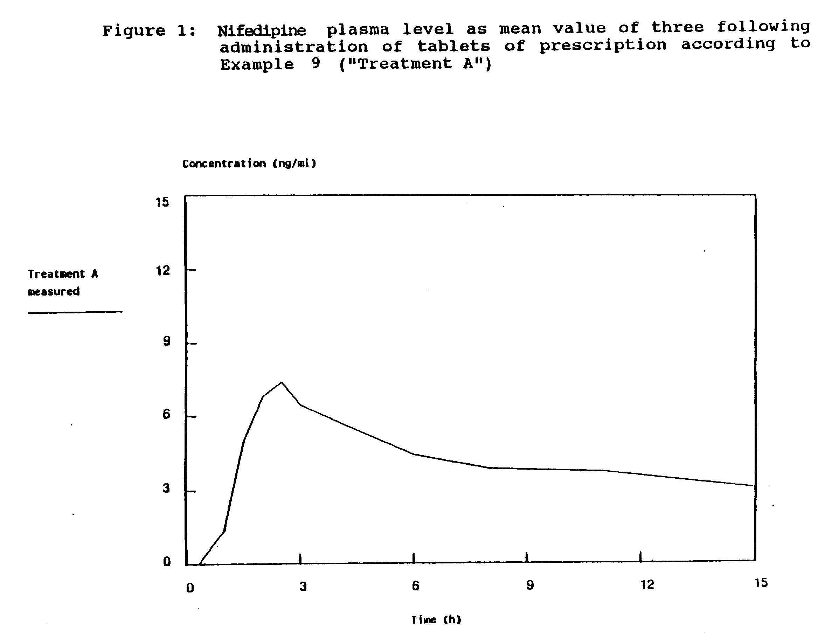

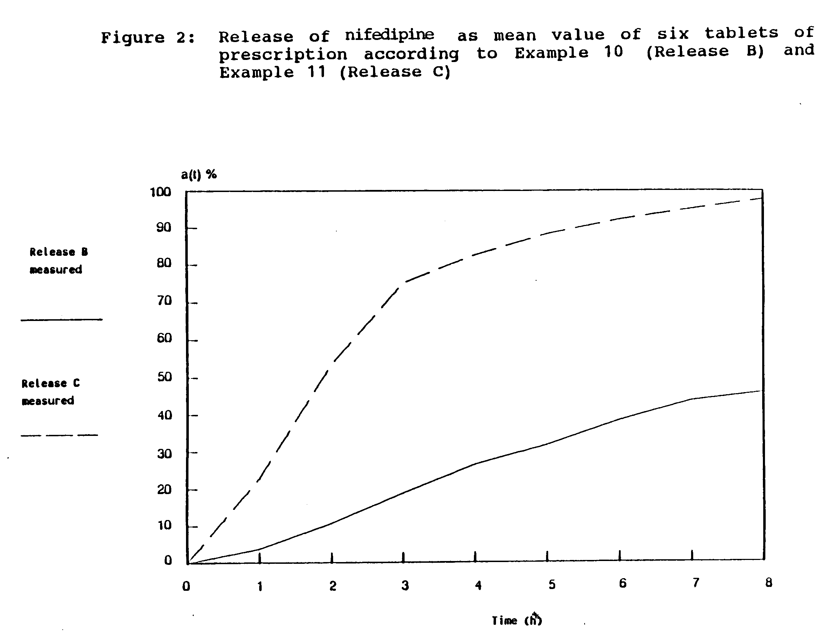

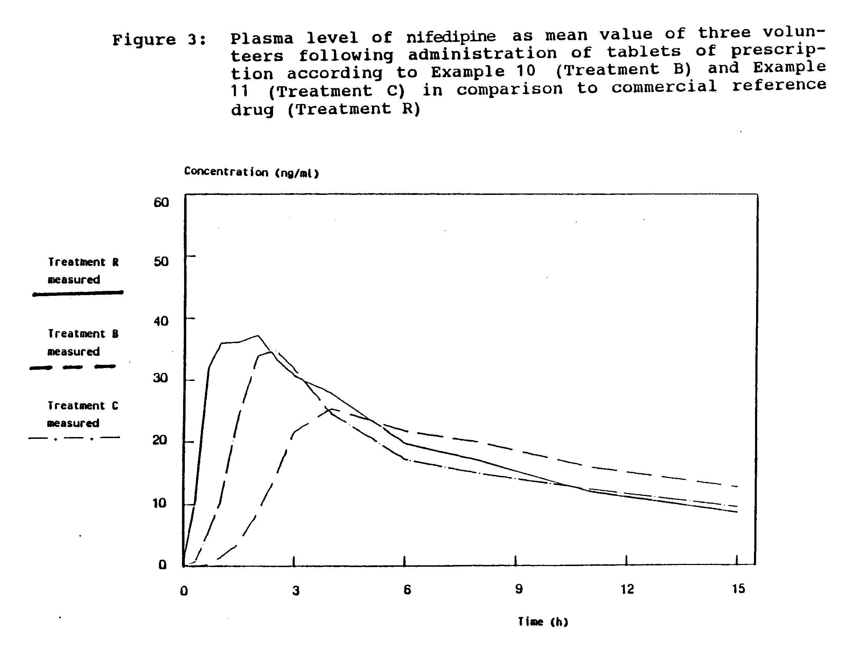

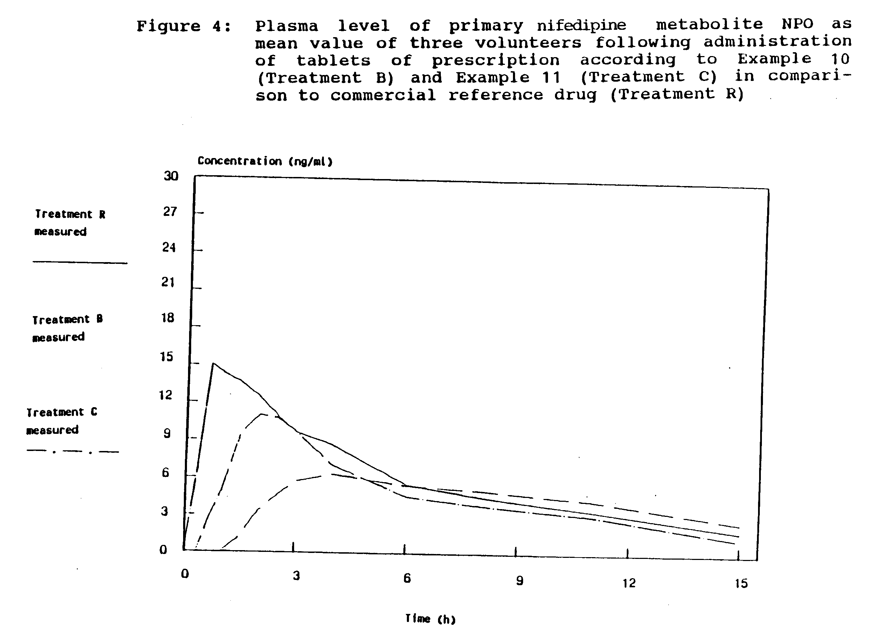

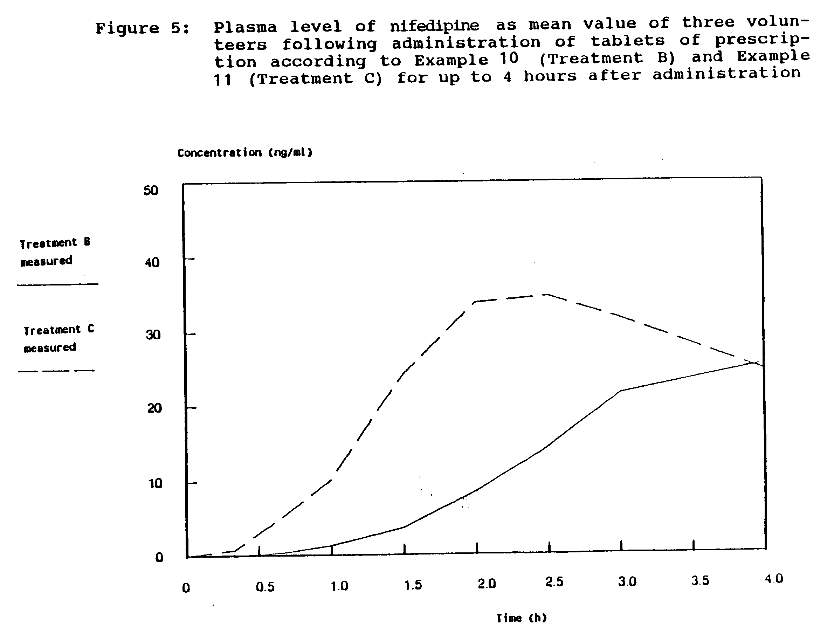

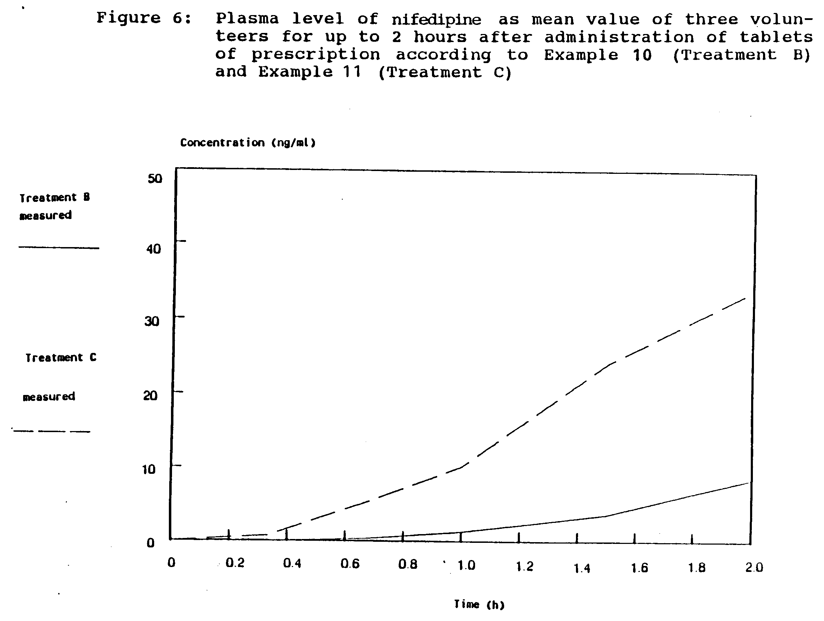

In the annexed drawing, Figure 1 shows the nifedipine plasma level as the mean value of three volunteers following the administration of tablets of the prescription according to Example 9 (called "Treatment A"); Figure 2 the release of nifedipine as the mean value of six determinations of tablets of the prescription according to Example 10 (called "Treatment B") and Example 11 (called "Treatment C"); Figure 3 the nifedipine plasma level as the mean value of three volunteers following the administration of tablets of the prescription according to Example 10 (called "Treatment B") and Example 11 (called "Treatment C") in comparison to a commercial reference product (called "Treatment R"); Figure 4 the plasma level of the primary nifedipine metabolite NPO as the mean value of three volunteers following the administration of tablets of the prescription according to Example 10 (called "Treatment B") and Example 11 (called "Treatment C") in comparison to a commercial reference product (called "Treatment R"); Figures 5 and 6 correspond to Figure 4 and show a time axis over 4 hours (Figure 5) and 2 hours (Figure 6) without the reference product.

In known erosion-controlled release systems, the release of low solubility pharmaceutical substances is governed by the slow dissolving of a suitable adjuvant, by the slow erosion of a gel or by the degradation of a digestable adjuvant. In the system of the invention, however, the erosion of such a system can be rendered controllable by adding a swelling controller. The swelling controller makes it possible to control the increase in volume of the adjuvants capable of swelling. The water or digestive fluid that comes into contact with the system is initially bound by the swelling controller. This means that a concentrated or saturated "solution" of the swelling controller is generated in the interior of the system. Thus the adjuvant capable of swelling is prevented from swelling there. In contrast, swelling controller dissolves in the outer region of the system. This means that there is enough water available here for the swelling of the adjuvant capable of swelling, and thus the pharmaceutical form begins to erode. This erosion moves from the exterior of the system to the interior thereof and is influenced by the ratio of swelling controller to adjuvant capable of swelling and by the presence of wetting influencers, e.g. hydrophobizing adjuvants. The system of the invention permits the use of adjuvants capable of limited swelling, such as microcrystalline cellulose and starch, which today are preferably used in rapid release oral preparations, in erosion-controlled pharmaceutical preparations.

The addition of adjuvants which influence the liquid exchange between the interior of the tablets and the surrounding volume, expands the possibilities of this invention for release control of the active agent. For instance incorporated wetting influencers (hydrophobizing or wetting agents) and soluble adjuvants offer additional ways of modulating the release pattern control of the pharmaceutical preparation. These adjuvants control the leaching out and thus the decrease in concentration of the swelling controller in the matrix by influencing the liquid exchange either positively or negatively.

As regards the solubility of the active agents, a "low solubility substance" means one to which over 30 mass parts of solvent (water) have to be added to dissolve 1 mass part thereof. Accordingly, to dissolve 1 mass part of a high solubility substance, less than 30 mass parts of solvent are required; see Arzneibuch der DDR, Akademie Verlag, Berlin 1985, I/II/4.0.

The person of skill in the art takes adjuvants capable of swelling to mean such substances that are capable of solvating and thus increasing in volume when they contact solvents, especially water or digestive fluids. Adjuvants of limited swelling capacity are characterised in that the increase in volume stops when the binding/cohesion forces in the molecule or molecule aggregate are equal to those that cause the increase in volume via the sorption/binding of water. In substances of unlimited swelling abilities, these forces result from the solvent interacting with macromolecules and are stronger than the binding/cohesion forces of the molecule/molecule aggregate. If enough solvating agent is present, the adjuvant capable of unlimited swelling will dissolve, thus causing the intermediate formation of a more or less pronounced gel phase.

Substances having higher affinity to water than the adjuvants capable of swelling are characterised in that if water is only available in a limited amount, e.g. in a saturated or highly concentrated (> 50%) solution of the swelling controller in water, they prevent the hydration of the adjuvants capable of swelling or dehydrate hydrated adjuvants capable of swelling.

It was surprising and could not be foreseen by the person of skill in the art that the procedure according to the invention would yield forms which display very slow and controlled erosion and do not, as was to be expected, degrade very rapidly and release the active agent for immediate resorption.

The process guarantees the complication-free preparation of the pharmaceutical preparation according to pharmaceutical and technological process steps known per se. The active agent release system according to the invention and the process for preparing the same ensure the optimal bioavailability of the incorporated active agents.

Specific examples of the low solubility active agents which are suitable for the release system are theophylline, nifedipine, prazosine, ibuprofen and carbamazepin. According to the teaching of this application, each of these active agents enables the preparation of advantageous erosion-controlled release systems if the adjuvants are used in the defined ratios.

Salbutamol, terbutaline and pholedrine are examples of high solubility active agents.

Microcrystalline celluloses such as the commercial products Heweten (VEB Freiberger Zellstoff- und Papierfabrik, Weißenborn, G.D.R.), Avicel (FMC Corp. Philadelphia, PA 19103), Elcema (Degussa, 6000 Frankfurt/M., F.R.G.) and Vitacel (J. Rettenmaier + Söhne, 7091 Holzmühle, F.R.G.) and starches are examples of adjuvants capable of limited swelling.

The preferred microcrystalline celluloses have a swelling ability of up to 100% of their volume in water. The preferred starches have a gel-forming tendency of less than 100% of the part of the volume which is swollen via water intake. More specifically, in water the starch exhibits a specific volume increase, and the volume of the gel formed thereby is less than this increase in volume (conditions: ambient temperatures, 22 to 25°C, determination after 24 h).

Known cellulose derivatives such as methyl cellulose, cellulose gum, hydroxypropyl cellulose and hydroxypropylmethyl cellulose are examples of adjuvants capable of unlimited swelling; see "Pharmazeutische Technologie", Ed. H. Sucker, Stuttgart 1978, p. 321 to 354.

The adjuvant having higher affinity to water than the prescription component capable of swelling will be referred to as swelling controller in this invention. Specific examples thereof are polyethylene glycols of various molar masses, preferably 400 to 35,000, or strongly hydrating (i.e. such that dissolve quickly and easily in water) inorganic compounds such as potassium sulfate, potassium chloride or sodium chloride.

The processing to tablets of the active agent release system of the invention is carried out according to conventional methods known to the person of skill in galenic art, e.g. blending the prescription components, direct tabletting or tabletting preceded by granulating. In this regard, the swelling controller can be present in the solution used for the granulation of the prescription components. Talc and conventional wetting influencers, e.g. hydrophobizing adjuvants such as fatty acids, metal salts of fatty acids and/or silicones, and wetting agents, are specific examples of tabletting adjuvants. These adjuvants also function as lubricants. They are present in an amount of 0 to 50 mass percent, preferably 0 to 10 mass percent, based on total mass.

The adjuvant capable of swelling and the swelling controller are present in a mass ratio of 1:3 to 100:1, preferably 1:1 to 50:1, especially 2:1 to 25:1, in the system of the invention. The ratio of the remaining components of the system (active agents and further adjuvants) to each other, to the adjuvants capable of swelling and to the swelling controller are not critical. They can be determined by the person of skill in the art in accordance with the practical requirements of preparation and therapy (dosage, pharmacokinetic profile, therapeutic targets).

The system of the invention can be present in the form of conventional formulations such as tablets or capsules (single unit drug dosage forms). It can also be a multicompartment form, or a part thereof, and, for example, be filled into a capsule. The multicompartment form means dividing the total dose into several small units (microforms such as microcapsules, pellets and microtablets; small microunits, usually having a size of under 3 mm, obtained by various preparation processes, e.g. coazervation, extrusion, compression, tabletting).

The following examples and comparative examples will explain the composition and the process for preparing the formulations according to the invention using theophylline as an example of a low solubility active agent. The tablets are prepared according to conventional tabletting processes.

### Comparative Example 1:

| | |
|---|---|
| Theophylline | 200.0 mg |
| Heweten 12* | 100.0 mg |
| Magnesium Stearate | 5.0 mg |

| | |
|---|---|
| *= microcrystalline cellulose | |

Release data (mean value of n = 5 determinations; determined according to MOLDENHAUER, KALA and ERBE, Pharmazie, Vol. 30, 1975, p.720):

| t[h] | 0 | 1 | 2 | 3 |
|---|---|---|---|---|
| at%¹ | 0 | 77.5 | 97.9 | 100.0 |
| s² | 0 | 5.95 | 1.05 | 1.02 |

| | | | | |
|---|---|---|---|---|
| ¹at% = the amount of active agent released from the pharmaceutical form after t hours, based on the incorporated dosis | | | | |
| ²s = standard deviation | | | | |

The tablets of Comparative Example 1 behave the way the person of skill in the art would expect them to. On account of the known degradation-promoting properties of the microcrystalline cellulose, the active agent is rapidly and completely released from the pharmaceutical preparation.

### Comparative Example 2:

| | |
|---|---|
| Theophylline | 200.0 mg |
| Heweten 12 | 20.0 mg |
| Magnesium Stearate | 5.0 mg |

Release data (as a mean value, n = 5):

| t[h] | 0 | 1 | 2 | 3 | 4 | 5 |
|---|---|---|---|---|---|---|
| at% | 0 | 29.0 | 54.3 | 78.7 | 93.5 | 100 |
| s | 0 | 3.23 | 8.63 | 8.0 | 3.98 | 0.48 |

If the amount of Heweten in the prescription is less, release will be decreased because the minimal solubility of theophylline is the predominant component in the release pattern of the active agent.
The person of skill in the art could not foresee that the addition of substances such as polyethylene glycols, which are used as solubilizers or to improve the solubility of low solubility active agents, would attain a delay in the active agent release of low solubility active agents from the systems as mentioned in Comparative Examples 1 and 2.

### Example 1:

| | |
|---|---|
| Theophylline | 200.0 mg |
| Heweten 12 | 100.0 mg |
| PEG 35000* | 5.0 mg |
| Magnesium Stearate | 5.0 mg |

| | |
|---|---|
| *PEG 35000 = polyethylene glycol of mean molar mass of 35000 | |

Release data (as a mean value, n = 5):

| t[h] | 0 | 1 | 2 | 3 | 4 | 5 | 6 | 7 |
|---|---|---|---|---|---|---|---|---|
| at% | 0 | 33.3 | 53.1 | 73.4 | 83.4 | 93.0 | 98.8 | 100.0 |
| s | 0 | 2.68 | 5.35 | 5.35 | 8.20 | 7.47 | 0.95 | 1.19 |

An increase in the amount of easily water-soluble PEG 35000 leads to a further delay in release.

### Example 2:

| | |
|---|---|
| Theophylline | 200.0 mg |
| Heweten 12 | 100.0 mg |
| PEG 35000 | 15.0 mg |
| Magnesium Stearate | 5.0 mg |

Release data (as a mean value, n = 5):

| t[h] | 0 | 1 | 2 | 3 | 4 | 5 | 6 | 7 |
|---|---|---|---|---|---|---|---|---|
| at% | 0 | 23.4 | 40.6 | 55.7 | 72.6 | 90.0 | 97.8 | 100.0 |
| s | 0 | 1.84 | 3.04 | 5.79 | 6.72 | 6.00 | 2.06 | 2.34 |

### Example 3:

| | |
|---|---|
| Theophylline | 200.0 mg |
| Heweten 12 | 100.0 mg |
| PEG 35000 | 50.0 mg |
| Magnesium Stearate | 5.0 mg |

Release data (as a mean value, n = 5):

| t[h] | 0 | 1 | 2 | 3 | 4 | 5 | 6 | 7 | 8 |
|---|---|---|---|---|---|---|---|---|---|
| at% | 0 | 15.5 | 24.5 | 37.3 | 46.5 | 58.2 | 66.4 | 74.3 | 79.5 |
| s | 0 | 1.02 | 1.35 | 3.86 | 6.95 | 8.60 | 10.0 | 11.1 | 5.55 |

When combined with a stronger hydrating adjuvant, even small amounts of microcrystalline cellulose capable of limited swelling can cause a delaying effect for low solubility active agents (see Comparative Example 2).

### Example 4:

| | |
|---|---|
| Theophylline | 200.0 mg |
| Heweten 12 | 20.0 mg |
| PEG 35000 | 5.0 mg |
| Magnesium Stearate | 5.0 mg |

Release data (as a mean value, n = 5):

| t[h] | 0 | 1 | 2 | 3 | 4 | 5 | 6 | 7 | 8 |
|---|---|---|---|---|---|---|---|---|---|
| at% | 0 | 22.6 | 33.6 | 44.8 | 56.7 | 74.1 | 93.1 | 98.7 | 100.0 |
| s | 0 | 1.01 | 2.15 | 4.15 | 5.07 | 5.43 | 5.49 | 0.54 | 0.48 |

It becomes very apparent that in contrast to what the person of skill in the art would have expected, the addition of polyethylene glycol does not lead to the accelerated release of theophylline, but rather to a further delay in release.

The influence of further adjuvants will be demonstrated using the exemplary combination of
- an adjuvant capable of limited swelling/a swelling controller/a wetting influencer

### Example 5:

| | |
|---|---|
| Theophylline | 200.0 mg |
| Heweten 12 | 100.0 mg |
| PEG 35000 | 5.0 mg |
| Magnesium Stearate | 0.0 mg |

Release data (as a mean value, n = 5):

| t[h] | 0 | 1 | 2 | 3 | 4 |
|---|---|---|---|---|---|
| at% | 0 | 27.5 | 58.6 | 87.1 | 100.0 |
| s | 0 | 1.81 | 1.95 | 3.26 | 0.91 |

### Example 6:

| | |
|---|---|
| Theophylline | 200.0 mg |
| Heweten 12 | 100.0 mg |
| PEG 35000 | 5.0 mg |
| Magnesium Stearate | 2.0 mg |

Release data (as a mean value, n = 5):

| t[h] | 0 | 1 | 2 | 3 | 4 | 5 | 6 |
|---|---|---|---|---|---|---|---|
| at% | 0 | 26.3 | 47.2 | 66.6 | 87.7 | 95.5 | 100 |
| s | 0 | 1.26 | 2.83 | 3.70 | 3.59 | 2.26 | 1.61 |

### Example 7:

| | |
|---|---|
| Theophylline | 200.0 mg |
| Heweten 12 | 100.0 mg |
| PEG 35000 | 5.0 mg |
| Magnesium Stearate | 10.0 mg |

Release data (as a mean value, n = 5):

| t[h] | 0 | 1 | 2 | 3 | 4 | 5 | 6 | 7 | 8 |
|---|---|---|---|---|---|---|---|---|---|
| at% | 0 | 19.9 | 27.7 | 39.1 | 51.6 | 62.6 | 73.4 | 85.3 | 97.5 |
| s | 0 | 0.71 | 1.97 | 1.54 | 1.87 | 1.56 | 2.67 | 2.89 | 3.02 |

### Example 8:

| | |
|---|---|
| Theophylline | 200.0 mg |
| Heweten 12 | 100.0 mg |
| PEG 35000 | 5.0 mg |
| Magnesium Stearate | 50.0 mg |

Release data (as a mean value, n = 5):

| t[h] | 0 | 1 | 2 | 3 | 4 | 5 | 6 | 7 | 8 |
|---|---|---|---|---|---|---|---|---|---|
| at% | 0 | 13.5 | 18.5 | 22.5 | 26.3 | 28.9 | 32.2 | 34.0 | 35.3 |
| s | 0 | 1.04 | 0.84 | 0.44 | 0.70 | 0.89 | 0.87 | 0.51 | 0.56 |

The effects of the addition of adjuvants capable of unlimited swelling will also be illustrated using the procedure according to the invention and the example of a nifedipine delayed release pharmaceutical preparation. For this purpose, tablets of the prescriptions in Examples 9, 10 and 11 were prepared in accordance with conventional expert procedures. Their release pattern was evaluated both in vitro and in vivo.

### Example 9:

| | |
|---|---|
| Nifedipine | 20.0 mg |
| Avicel PH 101 | 150.0 mg |
| Magnesium Stearate | 5.0 mg |
| PEG 35000 | 5.0 mg |

### Example 10:

| | |
|---|---|
| Nifedipine | 20.0 mg |
| Avicel PH 101 | 70.0 mg |
| Magnesium Stearate | 5.0 mg |
| Methyl Cellulose 400 | 5.0 mg |
| PEG 35000 | 10.0 mg |

Release data (as a mean value, n = 6):

| t[h] | 0 | 1 | 2 | 3 | 4 | 5 | 6 | 7 | 8 |
|---|---|---|---|---|---|---|---|---|---|
| at% | 0 | 3.74 | 10.7 | 18.9 | 26.5 | 31.7 | 38.3 | 43.6 | 45.8 |

### Example 11:

| | |
|---|---|
| Nifedipine | 20.0 mg |
| Avicel PH 101 | 55.0 mg |
| Magnesium Stearate | 5.0 mg |
| Methyl Cellulose 400 | 20.0 mg |
| PEG 35000 | 10.0 mg |

Release data (as a mean value, n = 6):

| t[h] | 0 | 1 | 2 | 3 | 4 | 5 | 6 | 7 | 8 |
|---|---|---|---|---|---|---|---|---|---|
| at% | 0 | 22.8 | 53.5 | 75.1 | 82.5 | 88.2 | 92.1 | 95.0 | 97.7 |

Example 9 shows that with the system of the invention, the in vivo release of the active agent from the pharmaceutical preparation can be delayed to such an extent that the plasma concentrations observed in three volunteers aged between 18 and 40 following a single dose are too low for effective therapy (for blood taking time and analytical procedure, see below).
- Figure 1:: Nifedipine plasma level following administration of tablets of the prescription according to Example 9 ("Treatment A")
In an open, randomized, three-way cross-over study, a comparison was made between the effect that changes in the prescription (changes in the amount of adjuvant capable of unlimited swelling) had on the time-dependent availability of the pharmaceutical agent, on the plasma concentration of the active agent nifedipine and, as a control, of its primary metabolite NPO were compared to those of a commercial delayed release drug of the same dosage after a single dose of one tablet (20 mg) in each case. The following preparations were used in the test plan and in Figures 3 to 6:
- Reference drug/"Treatment R":
   20 mg delayed release tablet (commercially approved drug)
   Amount of active agent 20 mg nifedipine
- Test drug/"Treatment B":
   Nifedipine retard 20 mg, tablets, prescription according to Example 10;
   Amount of active agent 20 mg nifedipine
- Test drug/"Treatment C":
   Nifedipine retard 20 mg, tablets, prescription according to Example 11;
   Amount of active agent 20 mg nifedipine
The quantitative determination of nifedipine and its primary metabolite NPO was carried out on the basis of the plasma using a specific, validated HPLC method, the lowest quantification level being 1 ng/ml of plasma. The test persons were three male volunteers aged between 42 and 47, all of whom completed the study according to instructions. In the open, randomized, three-way cross-over study, the volunteers were administered the nifedipine drug determined by the randomization plan on three different occasions, separated by two wash-out periods. Food intake was inhibited for ten hours before and up to four hours after each drug administration. Immediately prior to drug administration (pre-dose) and after 20, 40, 60, 90, 120, 150 minutes, 3, 4, 6, 8, 11 and 15 hours, venous blood samples of 10 ml were taken and plasma obtained to determine the concentration of nifedipine and NPO.
The curves of the mean plasma level of nifedipine (Figures 3, 5, 6) and the primary metabolite NPO (Figure 4) show that the pharmaceutical preparations according to the invention permit the controlled release of active agents in a desired and therapeutically advantageous region.

As illustrated by the above results, the incorporation of a soluble adjuvant capable of more pronounced swelling (in this case methyl cellulose 400, for instance) will accelerate release.

This result could not have been foreseen by the person of skill in the art (methyl cellulose exhibits a pronounced delaying effect, which is widely in use, both on its own and in combination with low solubility polymers by forming a gel layer which hampers diffusion) and emerges from a distinct loosening of the binding structures caused by the swelling and disintegration of the methyl cellulose. This is because if the amount of soluble and, in this case, gel-forming adjuvants is increased, it is possible to detect accelerated release and thus improved in-vivo availability.

The behaviour of the pharmaceutical preparations according to the prescriptions in Examples 10 and 11 will be illustrated in the following figures.
- Figure 2:: The release of nifedipine from tablets of the prescription according to Example 10 (Release B) and Example 11 (Release C).
- Figure 3:: The plasma level of nifedipine following the administration of tablets of the prescription according to Example 10 (Treatment B) and Example 11 (Treatment C) in comparison to a commercial reference drug.
- Figure 4:: The plasma level of the primary nifedipine metabolite NPO following the administration of tablets of the prescription according to Example 10 (Treatment B) and Example 11 (Treatment C) in comparison to a commercial reference product.

The controllable lag phase which occurs during the release of the active agent (clearly seen in Figures 5 and 6 where the plasma level course is shown on a different scale) can be used to advantage for specific ranges of application.
- Figure 5:: The plasma level of nifedipine following the administration of tablets of the prescription according to Example 10 (Treatment B) and Example 11 (Treatment C) for up to four hours after administration.
- Figure 6:: The plasma level of nifedipine for up to 2 hours after the administration of tablets of the prescription according to Example 10 (Treatment B) and Example 11 (Treatment C).

The release data of high solubility active agents are only slightly influenced by the principle of delayed release according to the invention. This opens up particularly advantageous fields of application for a number of indications.

Using the combination of theophylline and salbutamol as an example, the principle according to the invention and its advantages for controlling the release behaviour of two active agents of different solubility will be illustrated.

### Example 12:

| | |
|---|---|
| Theophylline | 250.0 mg |
| Salbutamol Sulfate | 9.6 mg |
| Avicel PH 101 | 60.0 mg |
| PEG 35000 | 10.0 mg |
| Magnesium Stearate | 10.0 mg |

Release data (as a mean value, n = 5):

### Salbutamol

| t[h] | 0 | 1 | 2 | 3 | 4 | 5 | 6 | 7 |
|---|---|---|---|---|---|---|---|---|
| at% | 0 | 50.7 | 70.5 | 88.6 | 97.7 | 98.7 | 99.8 | 100 |
| s | 0 | 1.74 | 2.61 | 0.66 | 2.71 | 0.75 | 0.26 | 0 |

### Theophylline

| t[h] | 0 | 1 | 2 | 3 | 4 | 5 | 6 | 7 | 8 | 9 |
|---|---|---|---|---|---|---|---|---|---|---|
| at% | 0 | 19.7 | 31.5 | 46.3 | 60.2 | 70.8 | 83.1 | 91.4 | 97.7 | 100 |
| s | 0 | 1.21 | 1.84 | 2.15 | 2.71 | 1.96 | 1.48 | 1.55 | 0.78 | 0 |

The high solubility active agent is already released to over 50% within the first hour. Salbutamol is almost totally released after four hours, whereas theophylline is evenly control-released under almost zero-order kinetics.

In principle, the aforedescribed systems can be used for various active agents. In addition to the active agents used in agricultural chemistry such as fertilisers, plant protectives, e.g. insecticides and growth regulators, it can be used with preference for pharmaceutical agents in the field of human and veterinary medicine.

## Claims

1. An erosion-controlled release system for pharmaceutically active agents containing at least one low solubility pharmaceutically active agent, optionally together with high solubility pharmaceutically active agents, having delayed release for the low solubility active agent components and, if incorporated, only slightly delayed release for the high solubility active agent components, characterized in that it is obtainable by formulating in a manner known per se the active agent(s) with at least one adjuvant capable of limited swelling and at least one adjuvant (swelling controller) having higher affinity to water than the adjuvant capable of limited swelling and, optionally, under the addition of further conventional adjuvants and compressing the formulation, except a formulation containing 25 % theophylline, 60 % microcrystalline cellulose and 15 % polyethylene glycol MW 20,000 and except a constant release aspirin tablet comprising from 85-95 weight percent of aspirin, from 1.5-5 weight percent of microcrystalline cellulose, from 1-10 weight percent of cellulose acetate phthalate, from 0.75-4 weight percent of a plasticizer, from 0.75-5 weight percent of corn starch, and from 0.5 to 2 percent of a lubricant.

2. The erosion-controlled active agent release system according to claim 1, characterized in that the adjuvant capable of limited swelling is a microcrystalline cellulose.

3. The erosion-controlled active agent release system according to claim 2, characterized in that the microcrystalline cellulose has a swelling capacity of up to 100 % of its own volume in water.

4. The erosion-controlled active agent release system according to claim 1, characterized in that the adjuvant capable of limited swelling is starch.

5. The erosion-controlled active agent release system according to claim 4, characterized in that the starch has a gel-forming tendency of less than 100 % of the part of the volume which is swollen via water intake.

6. The erosion-controlled active agent release system according to claim 1, characterized in that it contains a polyethylene glycol, preferably having a mean relative molar mass of 400 to 35,000 as a swelling controller.

7. The erosion-controlled active agent release system according to claim 1, characterized in that it contains at least one strongly hydrating, inorganic compound as a swelling controller.

8. The erosion-controlled active agent release system according to claim 7, characterized in that it contains potassium sulfate as a strongly hydrating, inorganic compound.

9. The erosion-controlled active agent release system according to any of claims 1 to 8, characterized in that it contains conventional hydrophobizing adjuvants as the further additives.

10. The erosion-controlled active agent release system according to claim 9, characterized in that it contains fatty acids, metal salts of fatty acids and/or silicones as the hydrophobizing adjuvants.

11. The erosion-controlled active agent release system according to any of claims 1 to 10, characterized in that it has a mass ratio of adjuvant capable of limited swelling to swelling controller of 1:3 to 100:1.

12. The erosion-controlled active agent release system according to any of claims 1 to 11, characterized in that it contains 0 to 50 mass percent of hydrophobizing adjuvant, based on the total mass.

13. The erosion-controlled active agent release system according to any of claims 1 to 12, characterized in that it is present in the form of tablets.

14. The erosion-controlled active agent release system according to claims 1 to 12, characterized in that it is present at least partially in a multicompartment form.

15. The erosion-controlled active agent release system according to any of claims 1 to 12, characterized in that it is present in microform.

16. A process for preparing the erosion-controlled active agent release system according to any of claims 1 to 15, comprising formulating the active agent(s) in a manner known per se with at least one adjuvant capable of swelling and at least one adjuvant (swelling controller) having higher affinity to water than the adjuvant capable of swelling and optionally under the addition of further adjuvants and compressing the formulation.

## Patentansprüche

1. Erosionsgesteuertes Freigabesystem für pharmazeutische Wirkstoffe, enthaltend mindestens einen schwerlöslichen pharmazeutischen Wirkstoff, gegebenenfalls mit leichtlöslichen pharmazeutischen Wirkstoffen, mit verzögerter Freisetzung für die schwerlöslichen Wirkstoffkomponenten und, falls eingearbeitet, nur wenig verzögerter Freisetzung für die leichtlöslichen Wirkstoffkomponenten, dadurch gekennzeichnet, daß es durch Formulierung in an sich bekannter Weise des bzw. der mit mindestens einem begrenzt quellfähigen Hilfsstoff und mit mindestens einem Hilfsstoff (Quellungscontroler) mit höherer Affinität zu Wasser als der begrenzt quellfähige Hilfsstoff und gegebenenfalls unter Zusatz weiterer üblicher Hilfsstoffe und Verpressen der Formulierung erhältlich ist, ausgenommen eine Formulierung, die 25% Theophilin, 60% mikrokristalline Cellulose und 15% Polyethlyenglykol MW 20000 enthält und ausgenommen eine Aspirin-Tablette mit konstanter Freisetzung, umfassend von 85 bis 95 Gew.% Aspirin, von 1,5 bis 5 Gew.% mikrokristalline Cellulose, von 1 bis 10 Gew.% Celluloseacetatphthalat, von 0,75 bis 4 Gew.% eines Weichmachers, von 0,75 bis 5 Gew.% Maisstärke und von 0,5 bis 2 % eines Gleitmittels.

2. Erosionsgesteuertes Wirkstoff-Freigabesystem nach Anspruch 1, dadurch gekennzeichnet, daß der begrenzt quellfähige Hilfsstoff eine mikrokristalline Cellulose ist.

3. Erosionsgesteuertes Wirkstoff-Freigabesystem nach Anspruch 2, dadurch gekennzeichnet, daß die mikrokristalline Cellulose eine Quellfähigkeit von bis zu 100% ihres Volumens in Wasser aufweist.

4. Erosionsgesteuertes Wirkstoff-Freigabesystem nach Anspruch 1, dadurch gekennzeichnet, daß der begrenzt quellfähige Hilfsstoff Stärke ist.

5. Erosionsgesteuertes Wirkstoff-Freigabesystem nach Anspruch 4, dadurch gekennzeichnet, daß die Stärke Gelbildungstendenz von weniger als 100% des durch die Wasseraufnahme gequollenen Volumenanteils aufweist.

6. Erosionsgesteuertes Wirkstoff-Freigabesystem nach Anspruch 1, dadurch gekennzeichnet, daß es als Quellungscontroler ein Polyethylenglycol, vorzugsweise mit einer mittleren relativen Molmasse von 400 bis 35000 enthält.

7. Erosionsgesteuertes Wirkstoff-Freigabesystem nach Anspruch 1, dadurch gekennzeichnet, daß es als Quellungscontroler mindestens eine stark hydratisierte anorganische Verbindung enthält.

8. Erosionsgesteuertes Wirkstoff-Freigabesystem nach Anspruch 7, dadurch gekennzeichnet, daß es als stark hydratisierte anorganische Verbindung Kaliumsulfat enthält.

9. Erosionsgesteuertes Wirkstoff-Freigabesystem nach einem der Ansprüche 1 bis 8, dadurch gekennzeichnet, daß es als weitere Zusätze übliche hydrophobierende Hilfsstoffe enthält.

10. Erosionsgesteuertes Wirkstoff-Freigabesystem nach Anspruch 9, dadurch gekennzeichnet, daß es als hydrophobierende Hilfsstoffe Fettsäuren, Metallsalze von Fettsäuren und/oder Silikone enthält.

11. Erosionsgesteuertes Wirkstoff-Freigabesystem nach einem der Ansprüche 1 bis 10, dadurch gekennzeichnet, daß es ein Massenverhältnis zwischen begrenzt quellfähigem Hilfsstoff und Quellungscontroler von 1:3 bis 100:1 aufweist.

12. Erosionsgesteuertes Wirkstoff-Freigabesystem nach einem der Ansprüche 1 bis 11, dadurch gekennzeichnet, daß es bezogen auf die Gesamtmasse 0 bis 50 Massenprozent hydrophobierende Hilfsstoffe enthält.

13. Erosionsgesteuertes Wirkstoff-Freigabesystem nach einem der Ansprüche 1 bis 12, dadurch gekennzeichnet, daß es in Form von Tabletten vorliegt.

14. Erosionsgesteuertes Wirkstoff Freigabesystem nach Anspruch 1 bis 12, dadurch gekennzeichnet, daß es mindestens zu einem Teil als Mehrkompartimentform vorliegt.

15. Erosionsgesteuertes Wirkstoff-Freigabesystem nach einem der Ansprüche 1 bis 12, dadurch gekennzeichnet, daß es als Mikroform vorliegt.

16. Verfahren zur Herstellung des erosionsgesteuerten Wirkstoff-Freigabesystems nach einem der Ansprüche 1 bis 15, umfassend die Formulierung in an sich bekannter Weise des bzw. der Wirkstoffe mit mindestens einem quellfähigen Hilfsstoff und mit mindestens einem Hilfsstoff (Quellungscontroler) mit höherer Affinität zu Wasser als der quellfähige Hilfsstoff und gegebenenfalls unter Zusatz weiterer zusätzlicher Hilfsstoffe und verpressen der Formulierung.

## Revendications

1. Système de libération à érosion contrôlée pour agents pharmaceutiquement actifs, contenant au moins un agent pharmaceutiquement actif à solubilité faible avec facultativement des agents pharmaceutiquement actifs à solubilité élevée, présentant une libération retardée en ce qui concerne les composants de l'agent actif à solubilité faible et, s'ils sont incorporés, une libération uniquement légèrement retardée pour les composants des agents à solubilité élevée, caractérisé en ce qu'on peut l'obtenir en formulant de manière connue en soi le ou les agents actifs avec au moins un adjuvant capable de gonflement limité et au moins un adjuvant (agent de contrôle du gonflement) présentant une affinité plus élevée pour l'eau que l'adjuvant capable de gonflement limité et facultativement avec addition d'autres adjuvants classiques, et en comprimant la formulation, à l'exception d'une formulation contenant 25% de théophylline, 60% de cellulose microcristalline et 15% de polyéthylèneglycol de masse molaire de 20 000 et à l'exception d'un comprimé d'aspirine à libération constante comprenant de 85 à 95% en poids d'aspirine, de 1,5 à 5% en poids de cellulose microcristalline, de 1 à 10% en poids d'acétophtalate de cellulose, de 0,75 à 4% en poids d'un plastifiant, de 0,75 à 5% en poids d'amidon de maïs et de 0,5 à 2% en poids d'un lubrifiant.

2. Système de libération d'agents actifs à érosion contrôlée selon la revendication 1, caractérisé en ce que l'adjuvant capable de gonflement limité est une cellulose microcristalline.

3. Système de libération d'agents actifs à érosion contrôlée selon la revendication 2, caractérisé en ce que la cellulose microcristalline a une capacité de gonflement atteignant 100% de son volume propre dans l'eau.

4. Système de libération d'agents actifs à érosion contrôlée selon la revendication 1, caractérisé en ce que l'adjuvant capable de gonflement limité est l'amidon.

5. Système de libération d'agents actifs à érosion contrôlée selon la revendication 4, caractérisé en ce que l'amidon a une tendance à la gélification inférieure à 100% de la partie du volume qui est gonflée par absorption d'eau.

6. Système de libération d'agents actifs à érosion contrôlée selon la revendication 1, caractérisé en ce qu'il contient un polyéthylèneglycol ayant de préférence une masse molaire relative moyenne comprise entre 400 et 35 000 comme agent de contrôle du gonflement.

7. Système de libération d'agents actifs à érosion contrôlée selon la revendication 1, caractérisé en ce qu'il contient au moins un composé inorganique fortement hydratant comme agent de contrôle du gonflement.

8. Système de libération d'agents actifs à érosion contrôlée selon la revendication 7, caractérisé en ce qu'il contient du sulfate de potassium comme composé inorganique fortement hydratant.

9. Système de libération d'agents actifs à érosion contrôlée selon l'une quelconque des revendications 1 à 8, caractérisé en ce qu'il contient des agents classiques conférant l'hydrophobie comme autres additifs.

10. Système de libération d'agents actifs à érosion contrôlée selon la revendication 9, caractérisé en ce qu'il contient des acides gras, des sels métalliques d'acides gras et/ou des silicones comme adjuvants conférant l'hydrophobie.

11. Système de libération d'agents actifs à érosion contrôlée selon l'une quelconque des revendications 1 à 10, caractérisé en ce qu'il présente un rapport massique entre l'adjuvant capable de gonflement limité et l'agent de contrôle du gonflement de 1:3 à 100:1.

12. Système de libération d'agents actifs à érosion contrôlée selon l'une quelconque des revendications 1 à 11, caractérisé en ce qu'il contient 0 à 50% en masse d'agent conférant l'hydrophobie, par rapport à la masse totale.

13. Système de libération d'agents actifs à érosion contrôlée selon l'une quelconque des revendications 1 à 12, caractérisé en ce qu'il est présent sous forme de comprimés.

14. Système de libération d'agents actifs à érosion contrôlée selon les revendications 1 à 12, caractérisé en ce qu'il est présent au moins partiellement sous une forme à compartiments multiples.

15. Système de libération d'agents actifs à érosion contrôlée selon l'une quelconque des revendications 1 à 12, caractérisé en ce qu'il est présent sous une microforme.

16. Procédé de préparation du système de libération d'agents actifs à érosion contrôlée selon l'une quelconque des revendications 1 à 15, comprenant la formulation du ou des agents actifs de manière connue en soi avec au moins un adjuvant capable de gonflement et au moins un adjuvant (agent de contrôle du gonflement) ayant une affinité plus élevée pour l'eau que l'adjuvant capable de gonflement et facultativement avec addition d'autres adjuvants et la compression de la formulation.
